# EUROPEAN PATENT SPECIFICATION

(11) **EP 1 433 378 B1**
(45) Date of publication and mention of the grant of the patent: **02.11.2011**
(21) Application number: 03078990.3
(22) Date of filing: 23.12.2003
(51) Int. Cl.: A01H 5/00

(54) **Resistance to powdery mildew infection and absence of necrosis in cucumber, Cucumis sativus**
Resistenz gegen echten Mehltaubefall und Abwesenheit von Necrosen in Gurke, Cucumis sativus
Résistance à l'infection de mildiou et absence de nécroses dans le concombre, Cucumis sativus

(30) Priority: 24.12.2002 NL 1022270
(43) Date of publication of application: 30.06.2004
(60) Divisional application: 10010808.3
(73) Proprietor: Nunhems B.V., 6083 AC Nunhems (NL)
(72) Inventor: Heuvelmans, Paul, 5988 KG Helden (NL)
(74) Representative: Krieg, Robert Alexander

(56) References cited:
- MOL, C.: "meeldauw en chlorose ontkoppeld" GROENTEN + FRUIT/GLASGROENTEN , vol. 25, 1992, pages 16-17, XP008018061
- ZIJLSTRA, S.: "resistentie and chlorose ontkoppeld" PROPHYTA, vol. 1, 1992, pages 22-25, XP008018074
- ZIJLSTRA S ET AL: "The relationship between powdery mildew (Sphaerotheca fuliginea) resistance and leaf chlorosis sensitivity in cucumber (Cucumis sativus) studied in single seed descent lines." EUPHYTICA, vol. 81, no. 2, 1995, pages 193-198, XP008018073 ISSN: 0014-2336
- GROOT S P C ET AL: "PHOSPHORUS NUTRITION AND SELECTION AGAINST LEAF CHLOROSIS RELATED TO POWDERY MILDEW RESISTANCE IN CUCUMBER" JOURNAL OF THE AMERICAN SOCIETY FOR HORTICULTURAL SCIENCE, vol. 117, no. 3, 1992, pages 500-503, XP008018079 ISSN: 0003-1062
- ZIJLSTRA, S.: "a new approach to the necrosis problem in mildew-resistant cucumbers / een nieuwe aanpak van het necroseproblem bij meeldauwresistente komkommers" PROPHYTA, vol. 41, no. 6, 1987, pages 138-140, XP008018059
- ZIJLSTRA SIERD ET AL: "Search for novel genes for resistance to powdery mildew (Sphaerotheca fuliginea) in cucumber (Cucumis sativus)." EUPHYTICA, vol. 64, no. 1-2, 1992, pages 31-37, XP008018058 ISSN: 0014-2336

## Description

### Field of the invention

The invention is in the field of cucumber breeding. In the northern European and Canadian winter crop of cucumbers, mildew resistant cucumber plants normally suffer from the development of necrosis when grown in the glasshouse during the winter months. Although cucumber plants exist which are not sensitive to the development of necrosis under these conditions, such plants do not have complete mildew resistance. It appears thus that a tight linkage between sensitivity to necrosis and mildew resistance exists. Despite descriptions in the literature that uncoupling of necrosis sensitivity and complete resistance to powdery mildew are believed to be impossible, the inventor has been able to create for the first time cucumber plants which have both complete mildew resistance and which are free of necrosis when grown under necrosis inducing conditions. In one embodiment of this invention a novel cucumber line, termed 'DC1', provides plants having both a complete mildew resistance and a complete absence of necrosis under necrosis inducing conditions. These plants are very valuable for the development of commercial cucumber varieties, which have the novel characteristics of DC1 and which enable optimal cultivation of glasshouse grown cucumber during the northern European and Canadian winter months.

### Background art

The present cucumber *(Cucumis sativus* L.) has been created by plant breeding from the initial crop gherkin, with which it has a strong phenotypic similarity. By plant breeding a new plant type has been developed in the current cucumber having exclusively female flowers. The cucumber fruit of this type develops from the non-fertilized flowers and is therefore parthenocarp. The current cucumber fruits contain just a few thin and empty seeds (these seeds are not viable, i.e., they cannot develop into a plant). Gherkin, cucumber, mini-cucumber, Beith Alpha and slicer belong to *C. sativus*. Courgette, melon, pumpkin and patisson belong together with cucumber to the family of *Cucurbitaceae.* Most current commercial cucumber varieties are hybrids.

One of the well known problems in cucumber production is the infestation of the plants by powdery mildew, caused by the fungus *Sphaerotheca fuliginea* (Schlecht. ex Fr.) and/or *Erysiphe cichoracearum* (DC. ex Mérat emend. Salm), which is generally referred to as "het wit" in Dutch.
After infection, the leaves, petioles, hypocotyl and/or the stem can show a pattern of white spots (fungal spores).

Infected plants may turn yellow, may be stunted and plants may eventually die. Fruits are hardly attacked. For most cucurbits, varieties are available which have some level of powdery mildew resistance. For example the greenhouse cucumber hybrid varieties Bella and Fidelio are resistant to mildew, while the hybrids Farbio and Corona are susceptible to mildew infection.

As early as 1969 the existing "Nederlands Instituut voor Veredeling van Tuinbouwgewassen" (IVT, the Dutch Institute for Breeding of Horticultural Crops) provided mildew resistant cucumber lines to the Dutch seed companies. Almost all of these companies have developed cucumber varieties with a good mildew resistance combined with a good value for culture and use. All of these varieties however show necrosis under low light intensity (e.g., between November and March in The Netherlands), which renders them unsuitable for the winter culture. Zijlstra et al. (1987) state that no-one has succeeded yet in obtaining a high level of mildew resistance in combination with an insensitivity for necrosis.

Further research of the IVT to new sources of mildew resistance that were possibly free of necrosis did not yield the expected results (Zijlstra en Groot, 1992; Mol, 1992). Yet it was shown that it was possible to select lines with a diminished susceptibility for necrosis and a limited level of mildew resistance from crossings between mildew susceptible and mildew resistant varieties (Zijlstra et al., 1995).

Each variety released and marketed up till now shows necrosis in the winter culture. This necrosis causes a strong decrease in fruit production, hence the production of mildew resistant varieties in the winter and early spring is economically not interesting.

Zijlstra et al. (1995) states that it is doubtful that complete mildew resistance can ever be combined with a complete absence of necrosis, after analyzing the mildew resistance present in the hybrid 'Profito' (completely mildew resistant) and the crossing of this mildew resistance in a line which is free of necrosis (but not resistant to mildew).

Zijlstra et al. (1992, 1995) found a positive correlation between mildew resistance and necrosis sensitivity. Zijlstra et al. concluded that one or more of the mildew resistance genes presumably cause necrosis sensitivity as a pleiotropic effect or are tightly linked to necrosis. "When one departs from coupling, then this seems not absolute and certainly for a large part can be separated from the mildew resistance genes. It seems like there are more genes involved (.....) of which some are strongly linked to the mildew resistance genes" (Zijlstra et al, 1992).
This detailed study states clearly that it is doubtful whether complete mildew resistance can ever be combined with a complete absence of necrosis. The use of extra phosphate in the liquid plant nutrients has improved the reliability of detecting the susceptibility for necrosis (Groot et al. 1990, 1992).

At present all available cucumber varieties can be grouped in 3 classes on the basis of their mildew resistance and necrosis sensitivity (the necrosis sensitivity has been measured under necrosis inducing conditions, such as short days and low light intensity typical for the winter production circumstances in glasshouses in Northern Europe or Canada, especially during the period between November and March, and with a high phosphate content in the plant nutrients):
(1) Susceptible to mildew with absence of necrosis, e.g., the varieties Sabrina, Ventura and Nicola;
(2) Partially resistant to mildew with absence of necrosis, e.g., Flamingo and Enigma;
(3) Completely mildew resistant but with necrosis, e.g., Cumlaude, Mistral, Savanna, Bella, Aramon and Belissima.

Until now there no cucumber plants combining complete mildew resistance with complete absence of necrosis under necrosis inducing conditions are publicly available.

### Summary of the invention

The invention concerns the breaking of the linkage between mildew resistance and necrosis sensitivity in the winter cultivation and the creation of cucumber plants combining a complete mildew resistance with a complete absence of necrosis in the winter cultivation of cucumber.

The invention provides also plants, seeds, plant cells and plant tissue of the species *Cucumis sativus L.* in which the characteristic of mildew resistance and the characteristic of absence of necrosis under necrosis-inducing conditions have been combined. Such necrosis inducing conditions are typically short days, low light intensity and a high phosphate concentration in the substrate (soil or other medium), which conditions are specifically found in the cultivation in the greenhouse in Northern Europe or Canada during the months November to February or March.
In one embodiment of the invention, the necrosis inducing conditions are the conditions in winter cultivation of cucumbers in glass houses in Northern Europe (including but not limited to France, Belgium, the Netherlands, Germany, Denmark, UK, and Sweden) or Canada, particularly heated winter cultivation as used in Northern Europe or Canada.

Another embodiment of the invention provides the DC1 plants, seeds, plant cells plant tissue and any plant derived from DC1 by asexual propagation or cloning, by selfing of DC1 or by crossing DC1 with other *Cucumis sativus L.* plants and selecting for the unique characteristics of DC1, namely the combination of complete mildew resistance with absence of necrosis in the winter cultivation. Especially preferred are plants having the characteristics of the plant deposited under deposit number NCIMB 41133, particularly a plant comprising the characteristics of complete resistance to powdery mildew and complete absence of necrosis under necrosis inducing conditions.

Also provided herein is any cucumber fruit, particularly a fruit with non-viable seed, harvested on any of the above plants and any use of such fruit in food preparations and the like.

Further disclosed are methods for transferring the combination of the characteristics of complete mildew resistance with the complete absence of necrosis to other *Cucumis sativus L.* plants, which lack either complete mildew resistance or complete absence of necrosis, or which lack both characteristics. This is done by crossing the new DC1 plants of the invention with such other plants, testing the progeny of such crossing for resistance to powdery mildew and for susceptibility to necrosis under necrosis inducing conditions, and selecting progeny plants which retain the combination of the complete mildew resistance and the absence of necrosis of DC1. Also included herein is a method for obtaining the cucumber plants of the invention, which method also comprises the step of obtaining doubled haploid cucumber plants containing the combination of said characteristics of mildew resistance and absence of necrosis under necrosis inducing conditions, and the resulting cucumber cells, tissues, plants and seeds contained in or obtained from such plants. Another method in accordance with this disclosure is the asexual propagation or cloning of the plants of the invention, containing the combination of said characteristics of mildew resistance and absence of necrosis under necrosis inducing conditions.

A further disclosure concerns the use of DC1 plants, or a plant of the DC1 line, as parent line for the production of hybrid cucumber varieties. A plant of the DC1 line can, for example, be used in a cross as a male or female parent line. The use of DC1 plants as a parent line for hybrid plant production is advantageous, because the uncoupling of mildew resistance and sensitivity for necrosis has been established in DC1.

The characteristics of complete absence of necrosis and mildew resistance can be transferred to other cucumber plants, such as other cucumber varieties, by traditional and well known breeding techniques in the art like crossing, back crossing and selfing (see Fehr, 1987 and Allard, 1960). In this way new varieties can be bred that combine good culture and agronomic value characteristics with complete resistance to mildew and complete absence of necrosis in the winter cultivation. Such cucumber varieties can significantly reduce the yield losses due to powdery mildew or the occurrence of necrosis in the winter (greenhouse) production of cucumber.

Also provided herein are the use of any one of the above plants as a parent in the production of cucumber plants or seeds. Such use comprises the step of selecting plants for absence of necrosis under necrosis inducing conditions. Such use produces hybrid plants or seeds.
Also provided are the use of any of the above plants to produce cucumber fruits, and the use of such plants to produce cucumber seed comprising the capacity to develop into a cucumber plant containing the characteristics of complete resistance for powdery mildew and complete absence of necrosis under necrosis inducing conditions.

Further provided is the use of a necrosis test under necrosis inducing conditions and a powdery mildew test to select cucumber plants with a complete absence of necrosis and a complete resistance to powdery mildew.

### Detailed description of the invention

The following definitions are presented for clarification of the invention and for interpretation of the description and the claims. These definitions are not meant to limit the scope of the legal protection for this invention.

### Definitions:

"Phenotype" is the observable external appearance of the plant as a result of the interaction between its genotype and its environment. It includes all observable morphological and physiological characteristics of the plant.

"Genotype" is the total of inheritable genetic information of a plant, which - partly influenced by environmental factors - is expressed in the phenotype in a certain manner.

"Cucumber plant" as used herein refers to a plant of the botanical species *Cucumis sativus* L. (like *Cucumis sativus* L. subspecies *sativus*). For references to cucumber fruits the specific term 'cucumber fruit' will be used herein. The term 'cucumber fruit' can also comprise gherkins, and long, medium or short cucumber fruits. The cucumber fruits eventually obtained at the harvest of the plants of the invention, including their use and processing thereof.

A "line" is a group of plants with a strong resembling genotype and phenotype. It can be formed by the progeny of one plant, after several generations of selfing or by vegetative propagation using plant cells, cell cultures, or tissue culture.
"DC1 line" refers to the DC1 plant as can be grown from the seeds deposited under NCIMB deposit number 41133, and each plant cell, cell culture, tissue culture, fruit, or seed thereof or derived there from, particularly such cells, fruit, or seed containing the characteristics of powdery mildew resistance combined with an absence of necrosis under necrosis inducing conditions.

A "plant variety" or "variety" as used herein, refers to a plant grouping within a single botanical taxon of the lowest known rank, which grouping, irrespective of whether the conditions for the grant of a breeder's right are fully met, can be defined by the expression of the characteristics resulting from a given genotype or combination of genotypes, can be distinguished from any other plant grouping by the expression of at least one of said characteristics and can be considered as a unit with regard to its suitability for being propagated unchanged.
The DC1 plants, as obtainable from the seeds deposited under NCIMB 41133, can be used to produce cucumber plants and to breed cucumber varieties with the characteristics of DC1.

"F1, F2, etc." refers to the consecutive, related generations following a cross between two parent plants or parent lines. The plants grown from the seeds produced by crossing two parent plants or lines is called the "F1" generation. Selfing the F1 plants results in the F2 generation, etc..

"Hybrids", "F1 hybrids" or "hybrid varieties" as used herein are terms to indicate the resulting seeds, or plants grown from those seeds, of the cross between two genetically different plants or parent lines.

"Mildew" or "powdery Mildew", as used herein, is a fungal disease of Cucurbitaceae plants, with particular symptoms in plant cells, tissue or organs of the plant caused by infection with a fungus of the genus *Sphaerotheca,* particularly the species *Sphaerotheca fuliginea* (Schlecht. ex Fr.) and/or of the genus *Erysiphe,* particularly the species *Ersysiphe cichoracearum* (DC. ex Mérat emend. Salm). Powdery mildew may be caused by infection of a single fungal species or by a combination of fungal species. Throughout this text, every reference to "mildew" for the cucumber plants of the invention or control cucumber plants, refers to "powdery mildew".

"Necrosis" of "necrosis symptoms" or "leaf necrosis" as being used herein is defined as the changes in the colour of the plant tissue observed under "necrosis inducing conditions", particularly in the necrosis test as described hereafter. The symptoms of necrosis show particularly an increasing yellowing and/or browning of particularly the leaf tissue, followed by the death of the tissue. The necrosis sensitivity, as used herein, is measured under necrosis inducing conditions.

As described hereafter, plants are classified on the basis of the necrosis test as either being "necrosis sensitive" or being "free from necrosis". The term " free of necrosis" will interchangeably be used herein with the terms "completely free of necrosis", "absence of necrosis" or "necrosis insensitive". The definition of necrosis does not include the reversible colour changes in the plant tissue, such as chlorosis, which do not have a genetic basis.

"Necrosis" as used here has, in contrast to chlorosis, a genetic basis. In the existing literature the terms "necrosis" and "chlorosis" are often confused. Chlorosis, however, is a tissue discoloration which is caused by environmental conditions, namely stress factors such as extreme temperatures, lack of water or nutrients, heavy fruit load etc., that disappears after elimination of the environmental cause.

Necrosis can be expressed in various ways: shrinkage of the leaf surface accompanied by a rounder form of the leaf and light yellow spots between the ribs, which later, in case of a great sensitivity, become necrotic (brown). The necrosis symptoms may occur in all degrees from just a light pigmenting of the leaf mass (often similar to chlorosis) to complete necrosis (death) of the leaf tissue.

"Necrosis inducing conditions" are conditions under which necrosis symptoms are induced (i.e., promoted, conduced, or stimulated), e.g., a high phosphate content (i.e., above 2.5 or 5 mM phosphate (as high as cucumber plants under normal illumination conditions still grow normally), particularly between 2.5 mM and 5 mM phosphate
(PO4³⁻), preferably ranging from 2.5 mM phosphate to 5 mM phosphate, particularly ranging from 4 mM to 5 mM phosphate in liquid nutrient, used to feed the plants), and/or low light conditions (3000 Lux or less), and/or a short day-length (8 hours of daylight or less), as typically found in northern Europe between November and March. It is obvious that the lowest amounts possible for the low light and short day conditions given above are those that still give an acceptable yield under normal practice to a person of ordinary skill in the art of cucumber cultivation. In one embodiment of this invention, "necrosis inducing conditions", as used herein, preferably are: 1) short days (8 hours of daylight or less), 2) low light intensity (3000 Lux or less) typical for the winter production circumstances in glasshouses in Northern Europe or Canada, especially during the period between November and March, and 3) a high phosphate content in the plant nutrients (higher then 2.5 mM).

### "Necrosis test"

The conditions as described in the "necrosis test" are one particular set of necrosis inducing conditions, which can be reliably used to test whether cucumber plants are necrosis sensitive or insensitive. Necrosis-sensitive control plants, such as for example Bella, will show necrosis symptoms in necrosis inducing conditions, like in the necrosis test. Control plants which are free of necrosis are also included in this test, e.g. , these serve as control that the plants to be tested are free of necrosis.

These plants are interplanted at random in the experiment, but any division or other planting methods can be used. Necrosis inducing conditions are typical for the winter production in heated glasshouses.

The necrosis test is carried out in the greenhouse between November and March, and is used to classify a line as "free of necrosis" or as "necrosis sensitive". Either soil-less culture (like stone wool) or soil culture may be used. The test is carried out in at least two, and preferably 3, independent replicates in the greenhouse. In each replication, at least 10 but preferably at least 20 plants of the line to be tested are grown together with at least 10, preferably at least 20, plants of at least one "necrosis sensitive" control variety; preferably together with at least 10, preferably at least 20, plants of at least one control variety which is "free of necrosis". Necrosis sensitive control varieties are for example Cumlaude, Mistral, Bella, Aramon, Savanna, Bellissima, and Sienna; necrosis free control varieties are for example Flamingo, Enigma, Sabrina, Nicola, and Ventura.

The test is only considered successful, if during the whole cultivation period, all leaves of all necrosis free control varieties remain free of necrosis symptoms (i.e., maintain green leaves), while the necrosis sensitive control varieties show necrosis symptoms. As soon as the necrosis sensitive control varieties, preferably plants with medium to some necrosis symptoms (scores 5 or 6 in the necrosis test described above), particularly Savanna or Sienna plants, show signs of necrosis in the test, all plants are screened for necrosis within three days.

Further it is disclosed that, all lines and control varieties are tested, as mentioned, according to the following protocol (as a person of ordinary skill in the art will understand, variations to these conditions are possible as long as necrosis is induced in susceptible control lines and the necrosis-free control lines remain free of necrosis):
- seeding between November 10 and December 20.
- cultivation of seedlings in an illuminated greenhouse with a temperature of 24-28 °C at seeding time to be lowered to 20 °C at the end of the seedling phase.
   Illumination: first 2 weeks 18 hours per 24 hours with 50 W/m² (± 3000 LUX).

After 14 days the illumination in the culture is brought back to 8 hours per 24 hours, this during about 14 days until the seedlings can be transplanted.
- planting period in the greenhouse is between December 15 and January 15 (in a non-illuminated greenhouse).
- nutrition: normal composition of nutrients for cucumber production, but with an absolute concentration of 5mM phosphate
- plant density: 1.4 plants/m²
- growing period: maximally 4 months after planting
- number of fruits per plant as is common in Dutch winter production: 3 ― 4 fruits at the stem from the 12^{th} leaf.
- plant form: normal umbrella or T-system (the umbrella system comprises: cutting the top of the plant at the wire and letting 2 side branches grow until a length of 60 cm; the T-system comprises: main stem without side branches until the wire ― height of 210 to 230 cm ―, with 2 primary side branches downwards followed by secondary side branches when the primary side branches are cut.
- observation of necrosis: this is done on the first real leaf within 3 days after the control varieties Savanna or Sienna show the first necrosis symptoms(approximately 80 days after seeding).
- observation for the first true leaf on a scale of 4 ― 7:
   7 = no necrosis symptoms
   6 = some necrosis in the leaves
   5 = medium symptoms of necrosis
   4 = complete necrosis

In this test, phosphate content in the nutrient medium, light intensity and the number of fruits per plant are critical factors that can influence the results of this test.

"Free of necrosis" or "completely free of necrosis" or "absence of necrosis " or "complete absence of necrosis" as used herein, means that no necrosis symptoms are seen on any of the leaves when testing the plants in the necrosis test as described above. This means that the plant tissue is green. "Free of necrosis", "completely free of necrosis", "absence of necrosis " or "complete absence of necrosis" refers to the obtaining (in test plants) of the same level of necrosis (meaning no symptoms), using standard necrosis test conditions, preferably using the above necrosis test (under necrosis inducing conditions), as in plants of control cucumber varieties that are free of necrosis, such as Flamingo, Sabrina, Nicola, Ventura, or Enigma, when necrosis sensitive control plants, preferably Cumlaude, Mistral, Bella, Aramon, Savanna, Bellissima, or Sienna, show necrosis symptoms. Such comparative test is done under the same experimental conditions for test and control plants.

"Early production or cultivation" or "winter production or cultivation" or "early winter production or cultivation", or "winter and early spring cultivation", as used herein, refers to the growing of plants in a greenhouse or another protective and heated environment during the winter and early spring period, particularly in the period between October 1 and March 31, particularly the period between November 1 and March 31, preferably the period between November 1 and March 1, preferably such period wherein cucumber plants sensitive to necrosis, especially cucumbers of the varieties Cumlaude, Mistral, Savanna or Sienna, show necrosis symptoms. This period is characterised by its short days (8 hours of daylight or less) and its limited natural light intensity. "winter" as used herein refers to the period starting one month before the meteorological winter period and ending one month after the meteorological winter period in a specific country, preferably such country is Canada or Northern Europe, particularly Northern Europe refers to France, Belgium, Germany, The Netherlands, Denmark, Sweden, and the United Kingdom. the winter period is from the first day of the month containing the start of the meteorological winter, until the last day of the month containing the end of the meteorological winter in a specific country.

With "heated production" the usual heated greenhouse production is being meant as known in the art, particularly the usual Dutch greenhouse production or a similar production in a heated and protective environment in other countries.

"Resistance to powdery mildew", or "mildew resistance" as used herein refers to the prevention of the development of fungi in the form of mycelium growth and sporulation on the plant. "Complete resistance to powdery mildew", as used herein, is the resistance to powdery mildew under standard assay conditions, preferably the conditions set our below for the mildew resistance test, which is of the same level as that observed in control plants of the cucumber varieties Cumlaude, Mistral, Bella, Aramon, Savanna or Sienna, preferably the varieties Savanna or Sienna. Such comparative test is done under the same experimental conditions for test and control plants.

A mildew resistance test has been developed to test both the resistance in the leaf and the hypocotyl. The test discerns 3 resistance levels in the plant.
1. Plant is "completely resistant" (standard control variety with this phenotype is Bella). "Complete resistance" of a plant means that this plant is "hypocotyl resistant" and "completely leaf resistant" as determined in the mildew resistance test described below.
2. Plant is "partially resistant" (standard control variety with this phenotype is Flamingo). "Partial resistance" of a plant means that the plant is "hypocotyl resistant" and "partially leaf resistant" as determined in the mildew test described below.
3. Plant is "completely susceptible" (Standard control variety with this phenotype is Ventura). A plant is "completely susceptible" if no "hypocotyl resistance" (completely susceptible) and no "leaf resistance" (completely susceptible) is present, as determined in the mildew test described below.

Note that a partial or a complete leaf resistance is always associated with a complete hypocotyl resistance. Leaf resistance without hypocotyl resistance does not occur, but hypocotyl resistance without leaf resistance does occur.

"Mildew resistance test" or "powdery mildew resistance test":

the mildew resistance test is carried out as follows (as a person of ordinary skill in the art will understand, variations to this test are possible as long as the (partially) mildew-resistant and mildew-susceptible control plants show the expected symptoms):

It is an (all year round) test on young plants in which the plantlets are being inoculated 3 times with spores of powdery mildew from the cotyledon-stage (this is immediately after the cotyledons have fully emerged from the seed). Observation of leaf resistance is done on the first real leaf on a scale of 0 to 5. Hypocotyl resistance is observed on a scale of 0 to 2. Control varieties are included in this test.

Each test is conducted in at least 2 replicates. In each replicate, at least 24 plants per line to be tested are used, together with at least 24 plants of a control variety, preferably plants of the control varieties Savanna and Sienna, and at least 12 plants of a completely resistant variety, preferably the control variety Bella, at least 12 plants of a partially resistant variety, preferably Flamingo, and at least 12 plants of a completely susceptible variety, preferably Ventura. The test will only be regarded as successful if the control varieties do not deviate from their expected phenotype.

The powdery mildew resistance test contains the following steps:
- seed of the lines or varieties to be tested is sown in and covered by moist vermiculite in a container of 40 x 40 cm; at a temperature of 28 °C (day and night) for 24 hours (illumination conditions are not relevant, this can be either in the complete darkness or with continuous illumination, in one embodiment continuous illumination is used).
- seedlings will be planted in soil or a suitable substrate after 4 days
- a fresh spore suspension of *Sphaerotheca fuliginea* (produced and multiplied on a susceptible variety, e.g., Ventura) is made in a concentration of a minimum of 100,000 spores per millilitre.
- the inoculate is applied on the test plants with a simple plant sprayer such that the leaves will be fully wet. The temperature after the inoculation is set at 20 °C for 24 hours. Normal illumination conditions are used, preferably at least 5000 Lux.
- 7 days after seeding, the first inoculation is done
- 9 days after seeding, the second inoculation is done
- 11 days after seeding, the third inoculation is done
- observations are done on 11 and 18 days after the first inoculation and approximately 14 days after the 3^{rd} inoculation by judging infestation on leaf and hypocotyl (i.e., observation of leaf resistance and hypocotyl resistance).

The observation of the mildew resistance in this mildew resistance test is done as follows:

| Observation on hypocotyl: | | |
|---|---|---|
| "hypocotyl resistant" | score 0 | No visible disease symptoms on the hypocotyl |
| "partially hypocotyl resistant" | score 1 | Some disease symptoms (light sporulation) visible on the hypocotyl |
| "hypocotyl susceptible" | score 2 | Many disease symptoms (many spots with sporulation, a lot of sporulation) visible on the hypocotyl. |

Observations on leaf: all observations are made on the first real leaf: mycelium growth, the number of infected spots on the leaf and the number of conidia spores per infected spots are important in the observation.

| Leaf observation: | | |
|---|---|---|
| "Completely resistant" | score 0-1 | Light mycelium growth without sporulation or very lightly infected small spots with a very low amount of spores per spot |
| "Partially resistant" | score 2-3 | Little to many infected spots, medium sporulation |
| "Completely susceptible or non-resistant" | score 4-5 | Many infected spots, strong sporulation |

Similar disease scores and methods of observation are known in the art and can also be applied in accordance with this disclosure. Important is the comparison to control plants which are cultivated under the same conditions as the test plants. Control plants that can be used for easy classification of the test plants, or that can function as a standard for these observations are: for "completely resistant": Bella, Aramon, Bellissima, Cumlaude, Mistral, Savanna, or Sienna; for "partially resistant": Flamingo or Enigma; and for "non-resistant": Sabrina, Ventura, or Nicola.

"DC1 plant(s)" or "DC1 line": cucumber plant(s) which have the combination of the characteristic "complete freedom of necrosis" and "complete mildew resistance" as assessed in the necrosis and mildew tests described above, and of which seed has been deposited under NCIMB 41133.
Seeds of the DC1 line have been deposited under the Budapest Treaty by Aventis CropScience N.V. (Jozef Plateaustraat 22, BE-9000 Gent, Belgium) on May 29, 2002 at the National Collections of Industrial, Food and Marine Bacteria (NCIMB, 23 St. Machar Drive, Aberdeen, AB24 3RY, Scotland) under deposit number NCIMB 41133.

Besides the combination of the characteristics "complete freedom of necrosis" and "complete mildew resistance", the plants of the deposited DC1 line, which is a uniform inbred line of Dutch long or greenhouse cucumber, have also the following characteristics: large, horizontal leaves and uniform almost smooth fruits with a short neck. The fruits of DC1 are larger and smoother than the fruits of Sabrina and lighter in colour compared to the fruits of Bellissima.

The two characteristics "complete freedom of necrosis" and "complete mildew resistance" can be transferred to other cucumber lines or cucumber varieties by crossing and further selection of plants (in the described necrosis and mildew tests).

### Further description of the invention

This invention concerns new plants of the species *Cucumis sativus L*., cucumber, which possess a combination of the characteristics "complete freedom of necrosis" and "complete mildew resistance", as can be tested in the above described necrosis and mildew tests. In one embodiment of this invention a novel cucumber plant called "DC1" has been produced which combines these two characteristics.

Powdery mildew is an important fungal pathogen on cucumbers. Especially in northern European countries, such as the Netherlands, Denmark and Sweden, mildew often causes severe infections on greenhouse grown cucumber plants, leading to significant yield losses and hence control measures such as fungicide treatments are necessary during cultivation.

Although two fungal species can cause mildew infections (*Erisyphe cichoracearum* and *Sphaerotheca fuliginea*), the most common mildew infection on greenhouse-grown cucumbers in the Netherlands is caused by infection of cucumber plants with *Sphaerotheca fuliginea*. *Erisyphe cichoracearum* is less common in the northern European areas where greenhouse cucumber cultivation occurs.

Until now, it is generally known in northern European countries that mildew resistance in cucumber is always associated with sensitivity to necrosis under necrosis inducing conditions, such as the short days with low light intensity during northern European late autumn, winter and early spring. Despite attempts to break this negative linkage by breeding procedures (Robinson 1978; Zijlstra et al., 1987, 1995), it has up to date been impossible to create a cucumber plant in which the mildew resistance is dissociated from the necrosis sensitivity.

The novel cucumber plants developed by the inventor combine for the first time complete mildew resistance with complete insensitivity for necrosis.

It is one object of the invention to provide cucumber plants with "complete freedom of necrosis" and "complete mildew resistance" in the winter cultivation. Such plants were developed by crossing plants of a commercial hybrid variety Bella with plants of a line developed in house (line 117) by the inventor. The F₁ plants of this cross were selfed for several generations, until in the F₆ generation a plant with the desired characteristics was developed and identified as such.

The line developed from this new cucumber plant has been named DC1 and is one of the possible embodiments of the invention. DC1 plants can further be cultivated or bred by methods known in the art (such as crossing, backcrossing, setting, etc.) to develop a cucumber variety particularly suited for the winter cultivation in the greenhouse, due to the combination of its characteristics of complete mildew resistance and complete freedom of necrosis with good agronomic characteristics, such as high yield.

Hybrid plants are characterised by the splitting up of their characteristics in the next generation. These characteristics are hence not genetically stable in hybrids. Methods for the development of hybrids are well known in the state of the art. For example the method as described in US Patent 4,822,949 may be used.

Plant cells, which can be stored and cultivated under *in vitro* conditions and can be regenerated to a normal cucumber plant that contains the combination of the characteristics complete mildew resistance with complete insensitivity for necrosis, form another embodiment of this invention. These plant cells contain the genetic information of the selected plant line and are well suited for multiplication, transformation with additional DNA, and long term storage. Transformation of such cells is standard in the state of the art, and by such transformation certain additional resistance genes, genes for production enhancement, etc. can be inserted in the plants of the invention. Known techniques for transformation of cucumber are among others *Agrobacterium* (*A. rhizogenes* or *A. tumefaciens*) transformation and regeneration thereafter, as described for instance in W088/02405, W090/03725 and US 6,084,152, and direct transfer of DNA, etc., as described for cucumber for instance in the European patent application 99302909.9 (European patent publication number 0 952 224), and US Patent US 6,015,942.

To this invention also belong plants, varieties, lines, derived varieties or lines, and hybrids of the species *Cucumis sativus*, which contain the same unique combination of DC-1 characteristics, i.e., complete mildew resistance and complete freedom of necrosis.

To this disclosure also belong doubled haploids of the species *Cucumis sativus,* which contain the same unique combination of DC-1 characteristics, i.e., complete mildew resistance and complete freedom of necrosis.

Double haploid cucumber plants may for instance be generated by the method described in published European patent application EP0374755 or in US patent 5,492,827.

F1 hybrid cucumber seeds and F₁ hybrid cucumber plants can be generated by methods known in the art or, for example, as described in US patent 4,822,949.

The following examples present one of the possible embodiments of the underlying invention, but are not offered as any limitation on the rights applied for under this application. These examples are purely for illustration purposes and cannot be used for interpretation or limitation of the scope of protection.

### Examples

### Example 1: Development of DC1.

The DC1 line has been developed form a cross between the public variety Bella and an internally developed line "117".

The F1 progeny plants of this cross, the F2 generation, have been selfed several times and the plants have been further selected in the necrosis and mildew resistance test as described above, until the DC1 plant has eventually been selected in the F6 generation. The DC1 line contains the novel combination of the characteristics "complete absence of necrosis" and "complete resistance" to mildew.

The generation of the DC1 line can be depicted by the following crossing scheme:

| Year | Material | Activity |
|---|---|---|
| 0 | Bella X 117 | Crossing |
| 1 | F1 plants | Selfing |
| 2 | F2 plants | Selecting of plants and selfing |
| 3 | F3 plants | Selecting of plants and selfing |
| 4 | F4 plants | Selecting of plants and selfing |
| 5 | F5 plants | Selection of plants and selfing |
| 6 | F6 plants | Selection of DC1 plant |

Under the conditions of an early greenhouse heated cultivation (necrosis test) the absence of necrosis was determined in the DC1 line.

After extensive research to the inheritance of mildew resistance and the absence of necrosis susceptibility it was determined that the DC1 plants always have a complete mildew resistance in the absence of any necrosis symptoms. Seeds of DC1 have been deposited at NCIMB (23 St. Marchar Drive, Aberdeen AB2 1 RY, Scotland, UK) on May 29, 2002, and were assigned the deposit number NCIMB 41133.

### Example 2: characteristics of DC1

In order to always secure the occurrence of necrosis in the chosen test conditions, enhanced absolute concentrations of phosphate were used in the research program. In all tests, always one or more mildew resistant varieties are used as comparison, such as Mistral, Cumlaude, Cordoba, etc.. Such varieties all showed clear necrosis symptoms. None of the cucumber varieties known in the art showed the combination of complete mildew resistance and complete absence of necrosis under winter cultivation.

In tests (different locations), several varieties have been compared to the DC1 line in a necrosis and a mildew resistance test, as described above. Each of these tests were carried out in a number of repetitions, in accordance with the method described above. These tests confirmed the new and unique combination of characteristics that have been genetically fixed in the DC1 line.

### (a) Necrosis and mildew tests on 2 Locations: Nunhem's Zaden in Haelen (The Netherlands) and 's Gravenzande (The Netherlands)

Details of the necrosis test (for the general description, see above):
- stone wool culture in a heated glass house
- 3 replicates in the greenhouse
- seeding date: November 20, 1998
- observation of the necrosis symptoms: March 1999
- growth in lighted greenhouse between 28°C at seeding to 20 °C at the end of the growth phase
- illumination of 18 hours per 24 hours (50 W/m²), last two weeks 8 hour per 24 hours
- plant date in the glasshouse: December 20
- cultivation time: maximum 4 months after planting (with increasing day length and light intensity), plant fruit load as in the Dutch heated greenhouse cultivation
- T-system of plant structure: main stem without side branches until the wire (height of 210 to 230 cm), with 2 downward primary side branches, followed by secondary side branches when the primary side branches have been cut.

| Results of necrosis test: | | | |
|---|---|---|---|
| Plant line or variety | Necrosis observation Scale 4-7 | Necrosis symptoms | Conclusion (plant phenotype) |
| DC1 | 7 | None | Completely free of necrosis |
| Cumlaude | 4 | Present | Necrosis susceptible |
| Mistral | 4 | Present | Necrosis susceptible |
| Savanna | 5-6 | Present | Necrosis susceptible |
| Sienna | 5-6 | Present | Necrosis susceptible |
| Flamingo | 7 | None | Completely free of necrosis |
| Enigma | 7 | None | Completely free of necrosis |
| Sabrina | 7 | None | Completely free of necrosis |

Details of the mildew resistance test (for a general description, see above):
- 24 plants per line or variety, per replicate
- 2 replicates in the greenhouse
- fungal inoculate multiplication on 50 plants of Ventura
- observation: on the first real leaf, approximately 14 days after the third inoculation

| Results of mildew test: | | | |
|---|---|---|---|
| Plant line or variety | Symptoms hypocotyl 0= no symptoms 1=some symptoms 2=many symptoms | Symptoms first leaf Score 0-1* Score 2-3 ** Score 4-5*** | Conclusion (plant phenotype) |
| DC1 | 0 | 0-1 | Completely resistant |
| Cumlaude | 0 | 0-1 | Completely resistant |
| Mistral | 0 | 0-1 | Completely resistant |
| Savanna | 0 | 0-1 | Completely resistant |
| Sienna | 0 | 0-1 | Completely resistant |
| Flamingo | 0-1 | 2-3 | Partially resistant |
| Enigma | 0-1 | | Partially resistant |
| Sabrina | 2 | 4-5 | Susceptible |

| | | | |
|---|---|---|---|
| *0-1 = light mycelium growth without sporulation (or very little spots with a very low number of spores per spot) | | | |
| **2-3=little to many spots, medium sporulation | | | |
| ***4-5=many spots, strong sporulation | | | |

### (b) Location NAK Tuinbouw, Roelofarendsveen (The Netherlands)

Necrosis test description (for general description, see above):
- soil culture in a glass house with soil heating (minimum of 18 °C), soil is steamed before the start of the test
- 10 plants per line or variety per replicate
- 3 replicates
- seeding date: November 23, 1998
- plant date: December 23, 1998
- necrosis observation: end March 1999
- seeding at 24 °C, planted out after 4 days
- cultivation temperature after planting out 22-23 °C; 21-22 °C after transplanting
- transplanting about 14 days after seeding; 18 plants per m²
- illumination of 18 hours per 24 hours (50W/m²), no extra illumination after planting
- plant fruit load: 3-4 fruits at the stem
- plant structure: umbrella system (cutting the top of the plant at the wire and letting 2 side branches grow along the wire, the side branches are cut when they reach a length of approximately 60 cm)

| Results of the necrosis test: | | |
|---|---|---|
| Plant line or variety | Necrosis symptoms | Conclusion (plant phenotype) |
| DC1 | None | Completely free of necrosis |
| Bella | Present | Necrosis susceptible |
| Aramon | Present | Necrosis susceptible |
| Bellissima | Present | Necrosis susceptible |
| Savanna | Present | Necrosis susceptible |
| Sienna | Present | Necrosis susceptible |
| Flamingo | None | Completely free of necrosis |
| Enigma | None | Completely free of necrosis |
| Sabrina | None | Completely free of necrosis |
| Ventura | None | Completely free of necrosis |
| Nicola | None | Completely free of necrosis |

Description for the mildew resistance test (for general description, see above):
- 24 plants per line or variety per replicate
- 2 replicates in the greenhouse
- inoculate multiplication on 50 plants of Ventura
- observations: on first real leaf, approximately 14 days after the third inoculation

| Results of the mildew resistance test: | | | | |
|---|---|---|---|---|
| Plant line or variety | Symptoms hypocotyl 0=no symptoms 1= some symptoms 2=many symptoms | Symptoms first real leaf Scores: 0-1, 2-3, 4-5 * | Phenotype first leaf/ Number of plants: Complete resistant-Partially resistant ― Susceptible | Conclusion (plant phenotype) |
| DC1 | 0 | 0-1 | 48 - 0 - 0 | Complete resistant |
| Bella | 0 | 0-1 | 24 - 0 - 0 | Complete resistant |
| Aramon | 0 | 0-1 | 24 - 0 - 0 | Complete resistant |
| Bellissima | 0 | 0-1 | 24 - 0 - 0 | Complete resistant |
| Savanna | 0 | 0-1 | 24 - 0 - 0 | Complete resistant |
| Sienna | 0 | 0-1 | 24 - 0 - 0 | Complete resistant |
| Flamingo | 0-1 | 2-3 | 0 - 24 - 0 | Partially resistant |
| Enigma | 0-1 | 2-3 | 0 - 24 - 0 | Partially resistant |
| Sabrina | 2 | 4-5 | 0 - 0 - 24 | Susceptible |
| Ventura | 2 | 4-5 | 0 - 0 - 24 | Susceptible |
| Nicola | 2 | 4-5 | 0 - 0 - 24 | Susceptible |

| | | | | |
|---|---|---|---|---|
| * the meaning of the scores is the same as in Example 2(a) above | | | | |

From these results it can be concluded that DC1 is the only line being free of necrosis and completely mildew resistant in the winter cultivation and that by this clear result it has been shown that the linkage between these two characteristics has successfully been broken.

### Example 3: Development of 'DC2', a new cucumber line with the combination completely of absence of necrosis and complete mildew resistance.

Seed of DC1 is sown together with seed of the cultivated cucumber variety Sabrina (necrosis free/mildew susceptible). The plants are grown following normal cucumber cultivation practices.

During flowering, DC1 plants are used as pollinator on Sabrina (motherplants). Sufficient crossings are made to ensure the harvest of approximately 100 F1 seeds.

The F1 seed is sown directly after harvest in order to produce F2 seed. The female flowering F1 plants are pollinated by the male flowering F1 plants by hand pollination or by using pollinating insects in a common compartment. The minimum number of 5 mother plants are used so that at least about 2.000 F2 seeds will be obtained (preferably sufficient crosses are made for obtaining at least about 10.000 F2 seeds). The F2 seed is then harvested.

Subsequently, from the F2 generation plants are selected which are completely free of necrosis and are complete mildew resistant.

The mildew resistance test is conducted as described above. Seed of parent lines (DC1, Sabrina) as well as of control varieties is seeded in the beginning of November, together with the accompanying F1 and F2 seed.

Of DC1, Sabrina, the control varieties and the F1, 3 replicates of at least 24 plants per replicate are sown, of the F2 at least 2000 seeds (going up to about 10,000 seeds) are sown. All plants are tested for mildew resistance (hypocotyl and leaf resistance) as described above. The parent lines and the reference varieties have the expected phenotype: DC1 plants and the mildew resistant control varieties are mildew resistant, Sabrina and the susceptible varieties are completely susceptible. The F1 plants are susceptible and the F2 plants are splitting for both hypocotyl and leaf resistance.

F2 plants with both hypocotyl and leaf resistance are selected and planted in the greenhouse for testing necrosis sensitivity as described above. Plants that remain free of necrosis are selected.

All plants with mildew resistance and absence of necrosis are selfed separately per plant. Since cucumber can be easily multiplied by cuttings, 5 cuttings per plant are taken. Self pollinations are done per plant separately and the F3 seed is harvested in spring.

Part of the harvested F3 seed is used to conduct a mildew test in the summer together with Sabrina, DC1, the F1 and the other control or reference varieties as described before. Only the completely resistant F3 plants are being selected, other plants are discarded.

The spare seed of the completely resistant plants is used in a necrosis test in winter, together with DC1, Sabrina the F1 and the usual reference varieties. The plants that are completely free of necrosis are selected and selfed per plant.

The selected pants that fulfil the requirement of the combination 'completely free of necrosis' and 'complete mildew resistance' will show a splitting in other characteristics. Hence, the spare seed of the selected plants is used to test the general value for cultivation and use in practice of the selected plants. The breeder selects the most suitable plants and selfs these further until the resulting lines have a sufficient genetic purity (selfing until at least F6).

### Cited references:

Allard RW (1960) Principles of Plant Breeding. John Wiley & Sons, New York.

Fehr WR (1987) Principles of Cultivar Development, Volume 1, Theory and Techniques, Collier Macmillan Publishers, London. ISBN 0-02-949920-8.

Groot et al. (1990) Leaf chlorosis in mildew resistant cucumbers. High phosphate application improves selection against susceptibility, Prophyta. v. 44(10) p. 318-320.

Groot et al. (1992) Phosphorus nutrition and selection against leaf chlorosis related to Powdery mildew resistance in Cucumber; Journal of the American Society for Horticultural Science v. 117 (3) p. 500-503.

Mol (1992) Meeldauw en chlorose ontkoppeld; Groenten + Fruit/ Glasgroenten no.25, p. 16-17.

Robinson RW (1978) Linkage Relationship of Genes for Tolerance to Powdery Mildew in Cucumber; Cucurbit Genetic Cooperative Report 1:11

Zijlstra et al. (1987) A new approach to the necrosis problem in mildew - resistant cucumbers. Prophyta 41 Nr 6, pp138-140.

Zijlstra en Groot (1992) Search for novel genes for resistance to powdery mildew (Sphaerotheca fuliginea) in Cucumber (Cucumis sativus); Euphytica Vol. 64, 1-2 p. 31-37.

Zijlstra et al. (1992) Resistentie en chlorose ontkoppeld; Prophyta 1, p. 22-25.

Zijlstra et al (1995) The relationship between powdery mildew (Sphaerotheca fuliginea) resistance and leaf chlorosis sensitivity in Cucumber (Cucumis sativus) studied in single seed descent lines; Euphytica Vol. 81 , 2, p. 193-198.

## Claims

1. A plant of the species *Cucumis sativus* L, **characterized by** the combination of the properties:
(a) complete resistance for powdery mildew of the fungus *Sphaerotheca fuliginea,* and
(b) complete absence of necrosis when cultivated under necrosis inducing conditions, wherein said complete resistance for powdery mildew means that the plant is both hypocotyl resistant and completely leaf resistant, as determined in the powdery mildew resistance test set forth in the description; and wherein complete absence of necrosis when cultivated under necrosis-inducing conditions means that no necrosis symptoms are seen on any of the leaves as determined in the necrosis test set forth in the description,
said complete resistance for powdery mildew and said complete absence of necrosis being obtainable from seed deposited under deposit number NCIMB 41133.

2. The cucumber plant according to claim 1, wherein said plant is a hybrid

3. The plant derived from a plant of claim 1 or 2 by asexual propagation or cloning, by selfing or by crossing with other *Cucumis sativus* L. plants, **characterized by** the combination of the properties:
(a) complete resistance for powdery mildew of the fungus *Sphaerotheca fuliginea,* and
(b) complete absence of necrosis when cultivated under necrosis inducing conditions, wherein said complete resistance for powdery mildew means that the plant is both hypocotyl resistant and completely leaf resistant, as determined in the powdery mildew resistance test set forth in the description; and wherein complete absence of necrosis when cultivated under necrosis-inducing conditions means that no necrosis symptoms are seen on any of the leaves as determined in the necrosis test set forth in the description,
said complete resistance for powdery mildew and said complete absence of necrosis being obtainable from seed deposited under deposit number NCIMB 41133.

4. A cucumber fruit of a plant of any one of claims 1 to 3.

5. The fruit of claim 4, which contains only non-viable seeds.

6. Seeds of a plant of any one of claims 1 to 3, which when grown into a plant, contain the combination of said characteristics of complete resistance to powdery mildew and complete absence of necrosis in the mildew and necrosis tests set forth in the description.

7. A plant cell of a plant of any one of claims 1 to 3, which plant cell can be regenerated to a plant containing the combination of said characteristics of complete resistance to powdery mildew and complete absence of necrosis in the mildew and necrosis tests set forth in the description.

8. A plant tissue of a plant of any one of claims 1 to 3.

9. A plant cell obtainable from the cucumber seeds deposited under deposit number NCIMB 41133, which plant cell after *in vitro* cultivation in the appropriate media regenerates into a plant with the combination of the characteristics complete resistance for powdery mildew of the fungus *Sphaerotheca fuliginea* and complete absence of necrosis under necrosis inducing conditions, wherein said complete resistance for powdery mildew means that the plant is both hypocotyl resistant and completely leaf resistant, as determined in the powdery mildew resistance test set forth in the description; and wherein complete absence of necrosis under necrosis-inducing conditions means that no necrosis symptoms are seen on any of the leaves as determined in the necrosis test set forth in the description.

## Patentansprüche

1. Pflanze der Art *Cucumis sativus* L., **gekennzeichnet durch** die Kombination der Eigenschaften:
(a) vollständige Resistenz gegen Echten Mehltau des Pilzes *Sphaerotheca fuliginea,* und
(b) vollständiges Fehlen von Nekrose bei Kultivieren unter nekroseinduzierenden Bedingungen, wobei vollständige Resistenz gegen Echten Mehltau bedeutet, dass in dem in der Beschreibung angegebenen Echten-Mehltau-Resistenztest die Pflanze sowohl hypokotylresistent als auch vollständig blattresistent ist; und wobei vollständiges Fehlen von Nekrose bei Kultivieren unter nekroseinduzierenden Bedingungen bedeutet, dass gemäß dem in der Beschreibung angegebenen Nekrosetest an keinem der Blätter Nekrosesymptome beobachtet werden,
wobei die vollständige Resistenz gegen Echten Mehltau und das vollständige Fehlen von Nekrose von unter der Hinterlegungsnummer NCIMB 41133 hinterlegtem Saatgut erhältlich ist.

2. Gurkenpflanze nach Anspruch 1, wobei es sich bei der Pflanze um einen Hybrid handelt.

3. Pflanze, die von einer Pflanze nach Anspruch 1 oder 2 durch asexuelle Vermehrung oder Clonierung, durch Selbstung oder Kreuzen mit anderen *Cucumis sativus* L. Pflanzen abstammt, **gekennzeichnet durch** die Kombination der Eigenschaften:
(a) vollständige Resistenz gegen Echten Mehltau des Pilzes *Sphaerotheca fuliginea* und
(b) vollständiges Fehlen von Nekrose bei Kultivieren unter nekroseinduzierenden Bedingungen, wobei vollständige Resistenz gegen Echten Mehltau bedeutet, dass in dem in der Beschreibung angegebenen Echten-Mehltau-Resistenztest die Pflanze sowohl hypokotylresistent als auch vollständig blattresistent ist; und wobei vollständiges Fehlen von Nekrose bei Kultivieren unter nekroseinduzierenden Bedingungen bedeutet, dass gemäß dem in der Beschreibung angegebenen Nekrosetest an keinem der Blätter Nekrosesymptome beobachtet werden,
wobei die vollständige Resistenz gegen Echten Mehltau und das vollständige Fehlen von Nekrose von unter der Hinterlegungsnummer NCIMB 41133 hinterlegtem Saatgut erhältlich ist.

4. Gurkenfrucht einer Pflanze nach einem der Ansprüche 1 bis 3.

5. Frucht nach Anspruch 4, die nur nichtlebensfähige Samen enthält.

6. Samen einer Pflanze nach einem der Ansprüche 1 bis 3, die, wenn sie zu einer Pflanze herangezogen werden, die Kombination der Eigenschaften der vollständigen Resistenz gegen Echten Mehltau und des vollständigen Fehlens von Nekrose in den in der Beschreibung angegebenen Mehltau- und Nekrosetests enthalten.

7. Pflanzenzelle nach einem der Ansprüche 1 bis 3, die zu einer Pflanze enthaltend die Kombination der Eigenschaften der vollständigen Resistenz gegen Echten Mehltau und des vollständigen Fehlens von Nekrose in den in der Beschreibung angegebenen Mehltau- und Nekrosetests, regeneriert werden kann.

8. Pflanzengewebe einer Pflanze nach einem der Ansprüche 1 bis 3.

9. Pflanze, erhältlich von den unter der Hinterlegungsnummer NCIMB 41133 hinterlegten Gurkensamen, wobei sich die Pflanzenzelle nach invitro-Kultivieren in den entsprechenden Medien zu einer Pflanze mit der Kombination der Eigenschaften vollständige Resistenz gegen Echten Mehltau des Pilzes *Sphaerotheca fuliginea*, und vollständiges Fehlen von Nekrose bei Kultivieren unter nekroseinduzierenden Bedingungen, regeneriert, wobei vollständige Resistenz gegen Echten Mehltau bedeutet, dass in dem in der Beschreibung angegebenen Echten-Mehltau-Resistenztest die Pflanze sowohl hypokotylresistent als auch vollständig blattresistent ist; und wobei vollständiges Fehlen von Nekrose bei Kultivieren unter nekroseinduzierenden Bedingungen bedeutet, dass gemäß dem in dem Beschreibung angegebenen Nekrosetest an keinem der Blätter Nekrosesymptome beobachtet werden.

## Revendications

1. Plante de l'espèce *Cucumis sativus* L, **caractérisée par** la combinaison des propriétés :
(a) résistance totale pour l'oïdium du champignon *Sphaerotheca fuliginea,* et
(b) absence totale de nécrose lors de culture dans des conditions inductrices de nécrose, ladite résistance totale pour l'oïdium signifiant que la plante présente à la fois une résistance de l'hypocotyle et une résistance totale des feuilles, comme cela a été déterminé dans le test de résistance à l'oïdium énoncé dans la description ; et l'absence totale de nécrose lors de culture dans des conditions inductrices de nécrose signifiant qu'on ne voit aucun symptôme de nécrose sur aucune des feuilles comme cela a été déterminé dans le test de nécrose énoncé dans la description,
ladite résistance totale pour l'oïdium et ladite absence totale de nécrose
pouvant être obtenues à partir de graine déposée sous le numéro de dépôt NCIMB 41133.

2. Plante de concombre selon la revendication 1, **caractérisée en ce que** ladite plante est un hybride.

3. Plante issue d'une plante selon la revendication 1 ou 2 par propagation ou clonage asexué, par autofécondation ou par croisement avec d'autres plantes de *Cucumis sativus* L., **caractérisée par** la combinaison des propriétés :
(a) résistance totale pour l'oïdium du champignon *Sphaerotheca fuliginea,* et
(b) absence totale de nécrose lors de culture dans des conditions inductrices de nécrose, ladite résistance totale pour l'oïdium signifiant que la plante présente à la fois une résistance de l'hypocotyle et une résistance totale des feuilles, comme cela a été déterminé dans le test de résistance à l'oïdium énoncé dans la description ; et l'absence totale de nécrose lors de culture dans des conditions inductrices de nécrose signifiant qu'on ne voit aucun symptôme de nécrose sur aucune des feuilles comme cela a été déterminé dans le test de nécrose énoncé dans la description,
ladite résistance totale pour l'oïdium et ladite absence totale de nécrose
pouvant être obtenues à partir de graine déposée sous le numéro de dépôt NCIMB 41133.

4. Fruit de concombre d'une plante selon l'une quelconque des revendications 1 à 3.

5. Fruit selon la revendication 4, qui ne contient que des graines non viables.

6. Graines d'une plante selon l'une quelconque des revendications 1 à 3, qui, lorsqu'on les fait pousser jusqu'à l'obtention d'une plante, contiennent la combinaison desdites caractéristiques de résistance totale à l'oïdium et d'absence totale de nécrose dans les tests d'oïdium et de nécrose énoncés dans la description.

7. Cellule végétale d'une plante selon l'une quelconque des revendications 1 à 3, **caractérisée en ce que** ladite cellule végétale peut être régénérée en une plante contenant la combinaison desdites caractéristiques de résistance totale à l'oïdium et d'absence totale de nécrose dans les tests d'oïdium et de nécrose énoncés dans la description.

8. Tissu végétal d'une plante selon l'une quelconque des revendications 1 à 3.

9. Cellule végétale susceptible d'être obtenue des graines de concombre déposées sous le numéro de dépôt NCIMB 41133, **caractérisée en ce que** ladite cellule végétale, après culture *in vitro* dans le milieu approprié, se régénère en une plante ayant la combinaison des caractéristiques de résistance totale pour l'oïdium du champignon *Sphaerotheca fuliginea,* et l'absence totale de nécrose lors de culture dans des conditions inductrices de nécrose, ladite résistance totale pour l'oïdium signifiant que la plante présente à la fois une résistance de l'hypocotyle et une résistance totale des feuilles, comme cela a été déterminé dans le test de résistance à l'oïdium énoncé dans la description ; et l'absence totale de nécrose lors de culture dans des conditions inductrices de nécrose signifiant qu'on ne voit aucun symptôme de nécrose sur aucune des feuilles comme cela a été déterminé dans le test de nécrose énoncé dans la description.
